# EUROPEAN PATENT APPLICATION

(11) **EP 3 144 033 A1**
(43) Date of publication of application: **22.03.2017**
(21) Application number: 16189272.4
(22) Date of filing: 16.09.2016
(51) Int. Cl.: A61N 2/00, A61N 1/40

(54) **METHOD AND SYSTEM FOR CONFIGURING A MAGNETIC FIELD GENERATING DEVICE FOR DIRECTIONING MAGNETIC SUBSTANCES**

(30) Priority: 17.09.2015 BR 102015024044
(71) Applicant: Sociedade Beneficente Israelita Brasileira Hospital Albert Einstein, 05652-000 Sao Paulo - SP (BR)
(72) Inventor: Gamarra, Lionel Fernel, 05529-060 São Paulo - SP (BR); Mamani, Javier Bustamante, 06784-180 São Paulo - SP (BR); da Silva, Hélio Rodrigues, 04165-000 São Paulo - SP (BR); Aguiar, Marina Fontes de Paula, 05625-060 São Paulo - SP (BR); Souza, Taylla Klei Félix, 04938-160 São Paulo - SP (BR)
(74) Representative: Vossius & Partner Patentanwälte Rechtsanwälte mbB

(57) **Abstract**

The present invention relates to a method and to a system for configuring a magnetic field generating device (1) for directioning magnetic substances (300), comprising an image capturing device and a computer device, the image capturing device being electronically connected to the computer device, the image capturing device being configured to obtain at least one image of at least two dimensions of the body area (500) and locate, in said at least one image of two dimensions of the body area (500), a target area (400), the image capturing device generating at least one image of at least two dimensions of the target area (400) from said at least one image of at least two dimensions of the body area (500), the image capturing device being configured to send said at least one image of at least two dimensions of the target area (400) to the computer device, the computer device being configured to generate a three-dimensional geometry of the target area (400) from said at least one image of at least two dimensions of the target area (400), the computer device being further configured to generate a three-dimensional geometry of the magnetic field generating device (1) correlated to the three-dimensional geometry of the target area (400).

## Description

The present invention relates to a method and a system for configuring a magnetic-field generating device applied especially in non-invasive medical procedures. The device is used for treating tumors or different desired targets, and generates a magnetic field capable of directioning magnetic substances to the tumors or other targets of interest, without there being an invasive surgical procedure.

### Description of the prior art

Together with the development of mankind, which dates back to thousands of years, various genetic variations, mutations and new diseases have developed, which affect the human being. Often resulting from an out-of-time pace between evolution and human adaptation and a number of changes in habits, environments and cultures, there are various kinds of presently known tumors.

With the advance of the techniques and technologies in the field of medicine, to treat and fight disease and mutations, together with advances in related fields, such as physics and engineering, various methods have emerged for removing, treating and eliminating these affected regions (tumors).

In this area one points out Gliomas, which are the most usual tumors in the central nervous system. Among these, astrocytomas, including Glioblastoma Multiforme (GBM), are the most common and also the most aggressive ones, accounting for 76% of all Gliomas. The classification thereof is made according to the degree of malignancy, ranging from grade I to grade IV.

Astrocytomas of types I and II are tumors that grow slowly and may be present in the brain of patients for many years without symptomatic progression. On the other hand, astrocytomas of types III and IV are considered the most aggressive and malignant cancers. These cancers are known to emerge from cells of the glia and grow rapidly, reaching regions of the brain and spinal cord.

In spite of the expressive increase in basic and clinical studies over the last few decades, the average of survival of patients having high-grade glioma is of about one year, constituting one of the most devastating and lethal of all cancers that affect humans.

The prognosis is still quite limited, and the present-day treatment involves surgical intervention, besides sessions of chemotherapy and radiotherapy, in order to eliminate the infiltrating cells that invade the healthy tissue. However, the therapy employed in other neoplasia forms, such as total surgical removal of the organ or tumor with a margin of normal tissue may not be applied to brain tumors, since each brain region has a function that is vital to the organism.

Intravenous traditional chemotherapy has many toxic effects, and a low concentration of the drug crosses the hematoencephalic barrier. Passive biodistribution by systemic administration usually results in subtherapeutic doses in the tumor region, which not only does not lead to eradication of the lesion, but also may stimulate the growth and resistance of the malignant cells.

Another disadvantage is that chemotherapy is not selective for tumor cells, and increasing the dose may generate systemic toxicity. Besides, due to the high molecular heterogeneity of these tumors, they remain refractory to treatments in most cases.

In this context, works aiming at the development of innovative and effective approaches, such as refined Nanobiotechnology tools, for diagnose and treatment of brain tumors, are of the utmost relevance. Researches developed until now show different nanostructures involved in biomedical applications.

Among these are magnetic nanoparticles (magnetic drugs), which have exhibited an important function in the diagnose/therapy medicine of brain tumors by means of Magnetic Resonance Image analyses, enabling early detection of the disease, precise diagnoses and customized treatments, besides the capability of monitoring and the efficacy of localized treatments.

A promising approach has been quite exploited, by using an outer magnetic field to direction these nanoparticles to the tumor region and, as a result, optimize diagnose and/or therapy. This technique, called magnetofection, enables active directioning to the tumor, a potentially interesting strategy for gliomas, because it is not invasive and does not interfere with the normal function of the brain.

Among the factors that can influence the efficiency of the technique, one can cite the intensity of the magnetic field applied, the physicochemical properties of the magnetic nanoparticles, the stability thereof in the blood circulation and the administration route for administering these compounds.

One observes that, after the intravenous or intra-arterial administration of iron oxide nanoparticles, a static external magnetic field is applied for directioning these compounds to the tumor.

However, it is known that it is difficult to direct magnetic pharmaceuticals to the desired target area in an amount that enables them to fulfil their diagnostic or therapeutic function. In order to solve this problem, one has made formulations with specific binders on the surfaces of pharmaceuticals, with a view to reach the desired target area. However, there is no good availability of pharmaceutical in the desired target area, which results in low efficiency in their therapeutic and/diagnostic objective.

One of the ways to achieve good availability in the area of interest is to apply a static local magnetic field with high intensity and high magnetic field gradient, with a view to guarantee the accumulation of the magnetic pharmaceuticals in the target area and in an a larger amount as compared with all the attempts made to solve the problem. There are a few pieces of equipment that were developed, but that lack precision, because the geometry of the target is not taken into consideration. These pieces of equipment were developed without having the precision of the gradient of magnetic field generated, which is fundamental to the success of the technique.

One of the pieces of equipment is described in the US patent of invention US 7,723,311, which describes a piece of equipment to generate an external magnetic field, in order to conduct and release a substance containing nanoparticles to fight a determined damaged tissue (tumor). However, one observes that this US patent does not describe the possibility of altering the geometry of the apparatus magnet, such geometry being related to the geometry (morphology) of the target area.

Another solution developed is that described in US patent application no. US 2002/0147424. One observes that said application describes a system and a method for transporting drugs by the action of an electromagnet arranged in pads, the electromagnet carrying out magnetic procedures to transport said drugs to an area to be treated. This patent application discloses that the magnets of the pads are designed with edge-like geometry and may have variations only in their dimensions. Thus, one observes that said application does not disclose correlation between the magnet geometry and the geometry (morphology) of the target area to be treated.

A similar solution is described in US patent application no. US 2012/0265001, which describes a system for directioning magnetic nanoparticles through magnets for the treatment of tumors. This application describes specifically that the magnet is positioned at the exact location of the affected area, adjacent to it or still being implanted in the patient in the affected region, in order to attract the substance to the respective location of treatment. Thus, one observes that the geometry is standard, and one does not mention the possibility of correlating the magnet geometry to the geometry (morphology) of the affected area to be treated.

Another alternative to the invasive surgical procedure is disclosed in US patent application no. US 2012/0259155. This application describes an apparatus that conducts a drug by means of a magnet, which may receive a piece at its end, in order to optimize its magnetic field. One observes that, although the piece has different geometries, the latter are not related to the geometry (morphology) of the affected area to be treated. Moreover, said application does not disclose how such geometry is determined to be directioned to the permanent magnet.

One observes that none of the solutions known from the prior art describes a methodology to determine a magnet geometry of a magnetic field generating device, related to the target area. The magnet geometry of the magnetic field generating device having a magnetic field gradient capable of directioning and distributing pharmaceuticals bonded to nanoparticles to the target area for effective treatment.

### Objectives of the invention

A first objective of the present invention is to provide a magnetic field generating device capable of accumulating the pharmaceutical in the desired target area, taking into account the geometry (morphology) of the target area.

A second objective of the present invention is to provide a magnetic field generating device with maximum 3-Tesla intensity and that can be graduated in a precise manner, according to the needs of each case.

A third objective of the present invention is to provide a magnetic field generating device that has a magnetic field gradient achieved by simulating finite elements.

A fourth objective of the present invention is to provide a magnetic field generating device that enables one to control the target area where the pharmaceutical is to be accumulated, by varying a distance between the magnetic poles.

A final objective of the present invention is to provide a magnetic field generating device that is formed by a plurality of magnet modules modularly associated to one another, so as to achieve a magnetic field gradient suitable for a determined geometry (morphology) of the target area.

### Brief description of the invention

The objectives of the present invention are achieved by means of a method for configuring a magnetic field generating device to direction magnetic substances, which comprises the following steps:
i) obtaining at least one image of at least two dimensions of a body area;
ii) locating, in the at least one image of at least two dimensions of body area, a target area;
iii) generating, from said at least one image of at least two dimensions of the body area, at least one image of at least two dimensions of the target area;
iv) identifying and generating, from said at least one image of at least two dimensions of the target area, a three-dimensional geometry of the target area; and
v) generating a three-dimensional geometry of the magnetic field generating device, correlated to the three-dimensional geometry of the target area.

The objectives of the present invention are further achieved by means of a system for configuring a magnetic field generating device to direction magnetic substances, which comprises an image capturing device and a computer device, the image capturing device being electronically connected to the computer device, the image capturing device being configured so as to obtain at least image of at least two dimensions of the body area and to locate, in said at least image of at least two dimensions of body area, a target area, the image capturing device generating at least one image of at least two dimensions of the target area from said at least one image of at least two dimensions of body area, the image capturing device being configured so as to send said at least one image of at least two dimensions of target area to the computer device, the computer device being configured to generate a three-dimensional geometry of the target area from said at least one image of at least two dimensions of the target area, the computer device being further configured to generate a three-dimensional geometry of the magnetic field generating device correlated to the three-dimensional geometry of the target areas.

### Brief description of the drawings

The present will now be described in greater detail with reference to an example of embodiment represented in the drawings. The figures show:
Figure 1 illustrates the general assembly of the magnetic field generating device of the present invention;
Figure 2 illustrates the active part of the magnetic field generating device of the present invention;
Figure 3 illustrates the intensity of the magnetic field in the central line of the magnetic field generating device of the present invention, as a function of the current for air gaps with different dimensions (lengths);
Figure 4 illustrates the magnet modules, with a determined geometry, of the magnetic field generating device of the present invention;
Figure 5 illustrates different geometries for said at least one magnet module of the magnetic field generating device of the present invention and its respective simulations of static magnetic field gradient by the finite element method; and
Figures 6a and 6b illustrate images by magnetic resonance showing the efficiency of the magnetic field generating device of the present invention, figure 6a illustrating specifically the body area and the target area after administration of the magnetic pharmaceutical and prior to application of the magnetic field, and figure 6b illustrating specifically the body area and the target area after administration of the magnetic pharmaceutical and after application of the magnetic field.

### Detailed description of the invention

In order to overcome the problems faced by the techniques developed in the prior art, the present invention was developed. As said before, the present invention relates to a method and to a system for configuring a magnetic field generating device 1, used in non-invasive medical procedures.

More specifically, the magnetic field generating device 1 of the present invention is used to direction magnetic substances 300 to a target area 400 of a body area 500. The magnetic substances 300 have magnetic properties and may be constituted, either individually or in conjunction, by drugs, pharmaceuticals, medicaments and/or substances bonded to nanoparticles. It is noted that the magnetic substances 300 may be individually a "substance" or jointly a "composition". The magnetic substances 300 may have, in their composition, elements with magnetic properties, such as iron, nickel, cobalt and compounds thereof.

In a preferred embodiment, the magnetic substances 300 are used for treating diseases or conditions that affect the body area 500, as for example, tumors. The method and the system of the present invention may be used, for instance, for the treatment of tumors located in the brain, a region that often may not be operated easily by means of invasive surgical procedures. It is also possible to use the present method and system with contrast substances, usually employed in magnetic resonance examinations.

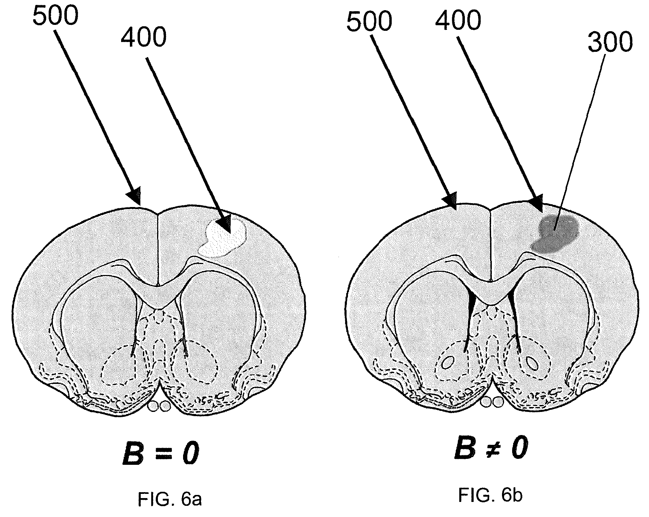

One observes that, regardless of the type of medical application, the magnetic substances 300 are directioned and distributed to the target area 400 of the body area 500, as can be observed in figure 6, by means of magnetic attraction between the magnetic substances 300 and the magnetic field generating device 1.

In a preferred embodiment, the magnetic substances 300 may be nanoparticles, which may be used either individually or bonded to drugs, medicaments and/or other substances, and the linkage may take place by means of covalent linkages or encapsulation thereof. The nanoparticles of the magnetic substances 300 used in the present invention are constituted by elements with magnetic properties, such as iron, nickel, cobalt and compounds thereof.

In a preferred embodiment, the nanoparticles of the magnetic substances 300 are constituted by iron oxide, more precisely magnetite (Fe₃O₄) or maghemite (γ-Fe₂O₃). In general, the nanoparticles contained in the magnetic substances 300 may be easily synthetized on laboratory scale.

Moreover, it is noted that the magnetic substances 300 have magnetic properties that cause them to interact with an external magnetic field, that is, they can be magnetized and attracted by the external magnetic field.

In the treatment of tumors, the magnetic substances 300 are administered/inserted into the patient's body. The administration may take place by intravenous or intra-arterial route, capable of directioning the drugs, pharmaceuticals, medicaments and/or substances bonded to nanoparticles contained in the magnetic substances 300 to the body area 500 for subsequent directioning by the present system and method.

Once the magnetic substances 300 have been administered, they are directioned by the magnetic field generating device 1 into the body area 500, in order to direction and distribute efficiently the magnetic substances 300 to the target area 400 (tumor).

In a preferred embodiment, the present invention provides a system having a magnetic field generating device 1, an image capturing device (not shown) and a computer device (not shown), which are electronically connected to each other.

Preferably, the image capturing device (not shown) is a magnetic resonance apparatus capable of generating images in up to at least two dimensions (two-dimensional or three-dimensional) of body area 500. Obviously, this is a preferred embodiment, and it is possible to use other devices such as apparatus for tomography, optical spectroscopy, ultrasound, among other equivalent methods to capture images of body areas 500.

One observes that the image capturing device (not shown) of the system of the present invention is configured to obtain at least one image of at least two dimensions of the body area 500. As mentioned before, the body area 500 may be any area of interest of the human body to be treated, such as the brain. Obviously, such a body area 500 represents only an example, and the present invention is not limited to this area alone.

Once at least one image of at least two dimensions of body area 500 is obtained, the image capturing device locates the target area 400. As mentioned before, the target area 400 is the specific location of the body area 500 to be treated, such as a tumor in the brain. Obviously such a target area 400 represents only an example, and the present invention is not limited to this area alone. For instance, the target area 400 may be other tumors or adipose tissue (for subsequent destruction thereof) and arteries (for subsequent removal of obstructions).

Later, from said at least one image of at least two dimensions of body area 500 and from the location of the target area 400, the image capturing device generates at least one image of at least two dimensions of target area 400, which is sent to the computer device.

Preferably, the computer device is an electronic device capable of receiving from the image capturing device said at least one image of at least two dimensions of the target area 400 and processing it correctly and generating from said at least one image of at least two dimensions of the target area 400, a three-dimensional geometry of the target area 400. As an example, the computer device may be a computer, a microprocessor, a microcontroller, among other devices capable of processing data.

Having received the geometry of the target area 400, the computer device makes a simulation of a mathematic modelling of application of a static magnetic field gradient in the target area 400. Such a modelling is achieved by simulation by the finite element theory and has the objective of determining the necessary intensity of magnetic field at determined locations of the target area 400, so that the magnetic substances 300 can be directioned, distributed and/or accumulated in said target area 400.

More specifically, the magnetic field gradient describes a relationship between distance between the magnets and the variation of the magnetic field. In other words, the magnetic field gradient establishes a relationship between the variation in the intensity of the magnetic field as a function of the distance between the magnets. In this way, the greater the variation in the intensity of the magnetic field, the better the precision of the directioning of the magnetic substances 300 will be.

As described before, the gradient generating process begins at the time of obtaining the three-dimensional geometry of the target area 400 generated by the computer device. Then, the three-dimensional geometry of the target area 400 is processed by means of a software installed on the computer device, as for example the software MATLAB®, which determines the borders of the three-dimensional geometry of the target area 400. The computer device is further configured to select the poles and generate the possible combinations of geometries for the magnetic field generating device 1.

Then, the data related to the poles and the possible combinations of geometries are sent to the modelling software of the computer device, such as a COMSOL Multiphysics® software, to parametrical simulation by finite elements of the magnetic potential of the mesh. The computer device is then configured to specify, through the software, contour conditions, mounting equations for the magnetic field generating device, selecting the mesh type, establishing parameters to refine the mesh, establishing the simulation precision, modelling the distribution of the magnetic field, leading the results to the geometry suggested by the software and determining the static magnetic field gradient.

Turning back to the example of the brain tumor, considering that the latter has geometry that is often heterogeneous, one observes that it may have variation in depth throughout its volume. In this regard, one observes that, upon applying the same intensity of magnetic field throughout the tumor volume in the brain, determined locations in the target area 400 may not accumulate efficiently the magnetic substances 300. For this reason, the static magnetic field gradient in the target area 400 should be generated.

From such modelling of the static magnetic field gradient, the computer device establishes a correlation between the geometry of the magnetic field generating device 1 and the three-dimensional geometry (morphology) of the target area 400, so that a preferred geometry for the magnetic field generating device 1 will be created.

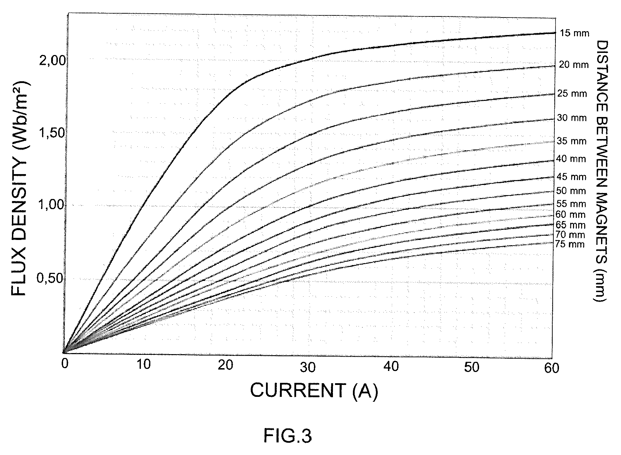

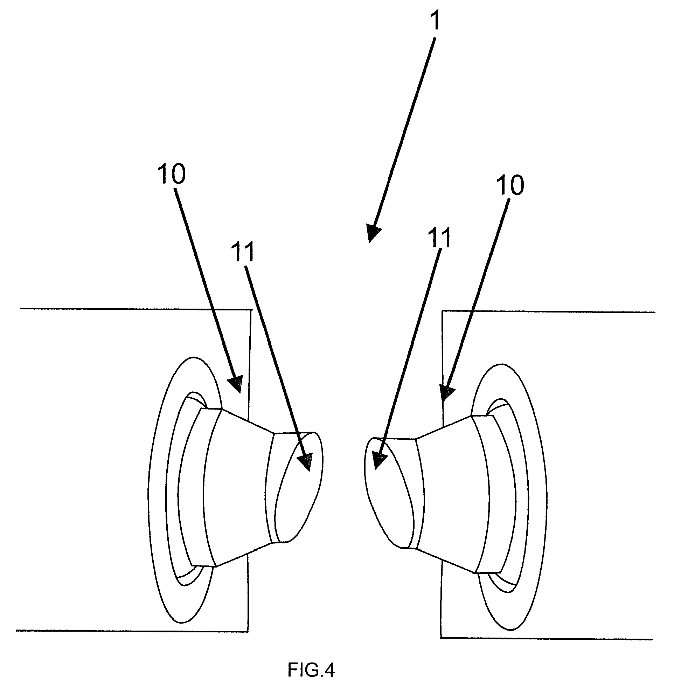

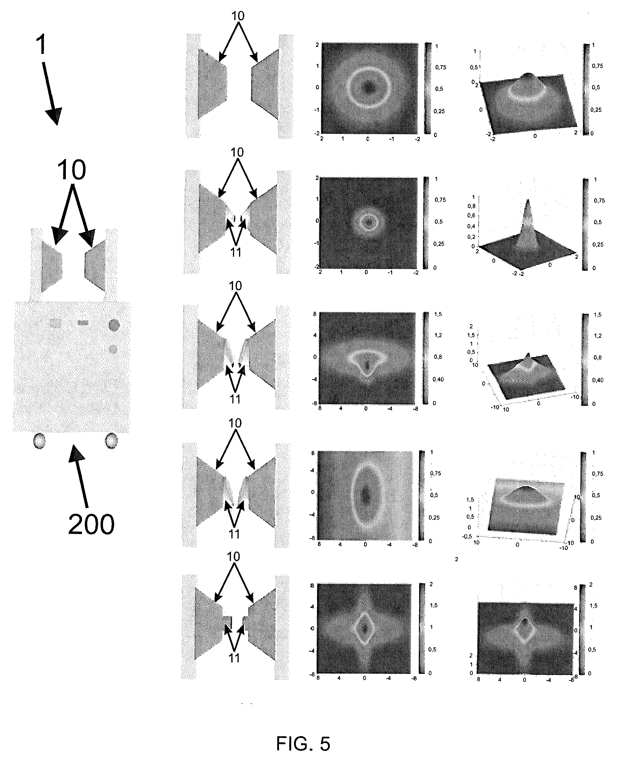

On the basis of the above-cited preferred geometry, it is possible to configure the magnetic field generating device 1. In a preferred embodiment, the magnetic field generating deice 1 may comprise at least one main magnet 10, the latter may comprise at least one magnet module 11, the magnet module 11 being made from low-carbon steel, as shown in figures 4 and 5. Said at least one magnet module 11 is made from ferromagnetic materials capable of generating a magnetic field upon application of an electric current. One observes that figure 3 illustrates the magnetic field intensity in the central line of the magnetic field generating device 1 of the present invention as a function of the current for air gaps with different dimensions (lengths).

In a preferred embodiment, said at least one magnet module 11 is made from low-carbon steel such as SAE 1010 steel, since the latter has lower saturation with an external magnetic field. It is also possible to use SAE 1020, SAE 1030 steels, or other materials with low-carbon characteristics.

One observes that, upon using a single magnet module 11, the latter is the main magnet 10 itself and is made so as to have geometry correlated to the three-dimensional geometry (morphology) of the target area 400 determined by the computer device. In this case, the single electric current is determined by the computer device and is applied to the main magnet 10, so that the latter will generate the static magnetic field gradient determined by the computer device.

If a plurality of magnet modules 11 are used, one observes that they are associated modularly to each other so as to form the main magnet 10, so that the main magnet 10 will have the geometry correlated to the three-dimensional geometry (morphology) of the target area 400 determined by the computer device. In this case, the plurality of magnet modules 11 may receive a plurality of electric currents, determined for each of the magnet modules of the plurality of magnet modules 11 by the computer device, so that the plurality of magnet modules 11 will generate different magnetic fields. In associating modularly the plurality of magnet modules 11 to form the main magnet 10, one observes that the latter, in conjunction, generate the static magnetic field gradient determined by the computer device.

In a preferred embodiment, the static magnetic field gradient is determined by the computer device for each point of the target area 400, so that magnetic fields of varying intensities will be applied at determined locations. Preferably, the static magnetic field gradient is intended to have varying magnetic fields of intensity of up to 3 Teslas and that may be graduated in a precise manner.

In a preferred embodiment, the magnetic field may reach the intensity of up to 3 Teslas. In this way, the higher the intensity of the magnetic field, the greater the precision of directioning of the magnetic substances 300 will be, thus optimizing the treatment of tumors of smaller size.

Considering that the static magnetic field gradient has a plurality of magnetic fields of varying intensity (gradient) at each location of the target area 400, one observes efficient directioning and distribution of the substances of magnetic properties 300 within the whole target area 400. This, in turn, provides greater efficiency in a subsequent hyperthermy process to be carried out for destruction of the target area 400.

Moreover, in a preferred embodiment, the magnetic field generating device 1 is interconnected to a power and command system to supply 1000 of the respective electric currents to at least one magnet module 11 of said at least one main magnet 10.

It should be noted that, upon varying the geometry of the target area 400, said at least one magnet module 11 of said at least one main magnet 10 should also be altered, that is, said at least one main magnet 10 is interchangeable, thus varying its geometry for each case. One observes in figure 5 examples of geometry for the main magnets 10 and their simulated magnetic field gradients.

One observes that said at least one main magnet 10 is mounted on a movable support base 200, which further has castors for moving the whole magnetic field generating device 1.

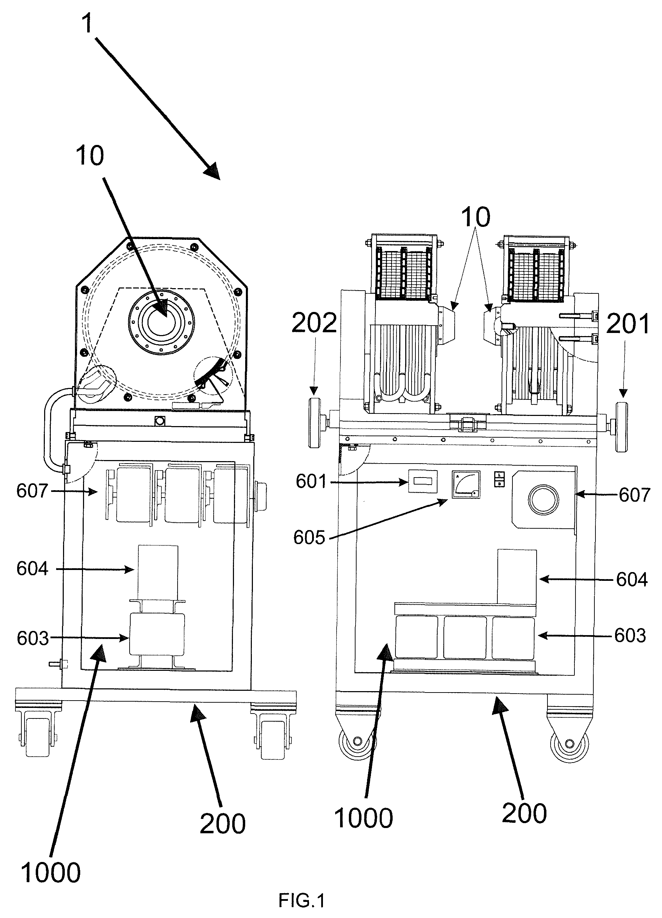

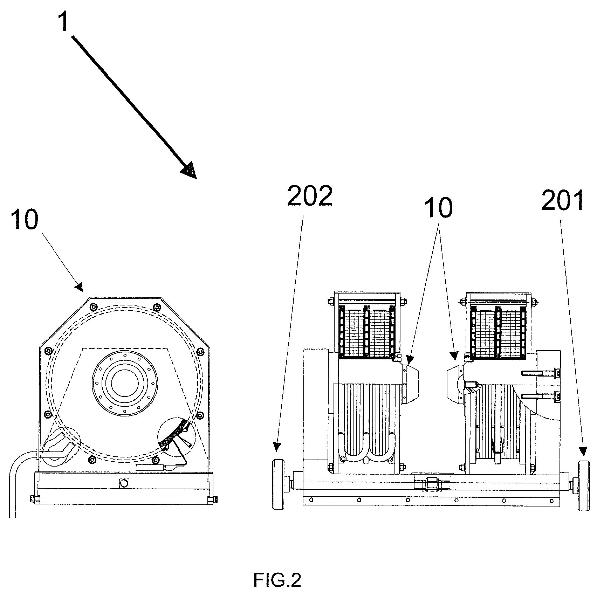

The movable support base 200 further has, at each end, at least one handle 201, 202, as shown in figures 1 and 2. One can observe that said at least one handle 201, 202 is mechanically associated to said at least one main magnet 10, so that said at least one main magnet 10 can move horizontally. This movement enables the distance with respect to the target area 400 of the body area 500 to be varied, such variation enabling more or less focus of the static magnetic field gradient on the target area 400.

For example, if said at least one handle 201, 202 is actuated to move said at least one main magnet 10 away from the target area 400 of the body area 500, one observes that the static magnetic field gradient will embrace most of or the whole of the target area 400. This enables the magnetic substances 300 to be directioned and distributed uniformly at all points of the target area 400.

In an opposite way, if said at least one handle 201, 202 is actuated to move said at least one main magnet 10 closer to the target area 400, one observes that the static magnetic field gradient will focus and embrace a smaller part of the target area 400. This enables the magnetic substances 300 to be directioned and distributed at specific points of interest of the target area 400.

Moreover, one observes that such a phenomenon is more efficient when the magnetic field generating device 1 comprises two main magnets 10. One observes that, in this case said at least one handle 201, 202 may be actuated individually or in conjunction. In a way similar to the above example, one observes that it is possible to move the two main magnets 10 closer or away, so that the magnetic substances 300 will be directioned and distributed, respectively, either to specific points of interest of the target area 400, or uniformly to all the points of the target area 400.

It is also possible that only one of the main magnets 400 to be moved by said at least one handle 201, 202, so that the gradient modelled by the computer device can be generated with respect to the target area 400.

In a preferred embodiment, the magnetic field generating device 1 is fed from a three-phase network. The power supply and command 1000 further has protective circuit-breakers 601, which turns off the supply in the event of overcharge on the magnetic field generating device 1. Additionally, the power supply and command system 1000 has a transformer 603 connected to rectifiers and command elements 604. The rectifiers and command elements 604 are further connected to an ammeter 605 and also to said at least one restrictive charge (not shown), so that the current value will be supplied to the user of the magnetic field generating device 1.

The magnet field generating device 1 further comprises a voltage shifter to adjust the excitation 607, which is configured to excite the device 1. Finally, the supply and command device 1000 further has a command button 608 for selectively turning on and off the magnetic field generating device 1.

Finally, one observes that the system for configuring a magnetic field generating device 1 is used by means of the following method to configure a magnetic field generating device 1:
i) obtaining at least one image of at least two dimensions of a body area 500;
ii) locating, in said at least one image of at least two dimensions of a body area 500, a target area 400;
iii) generating, from said at least one image of at least two dimensions of a body area 500, at least one image of at least two dimensions of the target area 400;
iv) identifying and generating, from said at least one image of at least two dimensions of the target area 400, a three-dimensional geometry of the target area 400; and
v) generating a three-dimensional geometry of the magnetic field generating device 1, correlated to the three-dimensional geometry of the target area 400.

Steps i) to iii) being carried out by means of an image capturing device and steps iv) and v) being carried out by means of a computer device.

The method further comprises a complementary step iv-a) for simulating a magnetic field gradient for three-dimensional geometry of the target area 400 between steps iv) and v).

The correlation in step v) to generate the three-dimensional geometry of the magnetic field generating device 1 is carried out from the simulation of the magnetic field gradient for three-dimensional geometry of the target area 400 carried out in the complementary step iv-a).

The method comprises a complementary step vi), after step v), for generating, from at least one magnet module 11, the geometry of the magnetic field generating device 1.

One observes that step vi) for generating the geometry of the magnetic field generating device 1 is made by means of a single magnet module 11 or by means of a modular association of a plurality of magnets 11. The single magnet module 11 presents the magnetic field gradient simulated in step iv-a). The plurality of magnets 11 of the magnetic field generating device 1 is modularly associated so as to have the magnetic field gradient simulated in step iv-a).

One observes in figure 6 the results when using the system and method of the present invention. Specifically, figure 6a illustrates the body area 500 after administration of the magnetic substances 300 and the target area 500 still not having, inside it, the magnetic substances 300. In figure 6b, one observes the result of applying the system and method of the present invention, where one views the concentration of magnetic substances 300 within the target area 400 of the body area 500.

After the directioning, distribution and accumulation of the magnetic substances 300 in the target area 400 of the body area 500, it is then possible to carry out the hyperthermy process to destroy the target area 400 in a much more efficient manner, without there being damage to the adjacent tissues out of the target area 400.

A preferred example of embodiment having been described, one should understand that the scope of the present invention embraces other possible variations, being limited only be the contents of the accompanying claims, which include the possible equivalents.

## Claims

1. A method for configuring a magnetic field generating device (1) to direction magnetic substances (300), **characterized by** comprising the following steps:
i) obtaining at least one image of at least two dimensions of a body area (500);
ii) locating, in said at least one image of at least two dimensions of the body area (500), a target area (400);
iii) generating, from said at least one image of at least two dimensions of the body area (500), at least one image of at least two dimensions of the target area (400);
iv) identifying and generating, from said at least one image of at least two dimensions of the target area (400), a three-dimensional geometry of the target area (400); and
v) generating a three-dimensional geometry of the magnetic field generating field (1) correlated to the three-dimensional geometry of the target area (400).

2. The method according to claim 1, **characterized in that** steps i) to iii) are carried out by means of an image capturing device and the steps iv) and v) are carried out by means of a computer device, the image capturing device being a magnetic resonance apparatus.

3. The method according to claim 1, **characterized in that** the body area (500) is a brain and the target area (400) is a brain tumor.

4. The method according to claim 1, **characterized by** comprising a complementary step iv-a) for simulating a magnetic field gradient for three-dimensional geometry of the target area (400) between steps iv) and v).

5. The method according to claim 4, **characterized in that** the correlation in step v) for generating the three-dimensional geometry of the magnetic field generating device (1) is carried out from the simulation of the magnetic field gradient for three-dimensional geometry of the target area (400) carried out in the complementary step iv-a).

6. The method according to claim 5, **characterized by** comprising a complementary step vi), after step v), for generating, from at least one magnet module (11), the geometry of the magnetic field generating device (1).

7. The method according to claim 6, **characterized in that** step vi) for generating the geometry of the magnetic field generating device (1) is made by means of a modular association of a plurality of magnet modules (11).

8. The method according to claim 7, **characterized in that** the plurality of magnet modules (11) of the magnetic field generating device (1) is modularly associated so as to generate the magnetic field gradient simulated in steps iv-a).

9. The method according to claim 7, **characterized in that** the magnetic substances (300) area selected from a group composed by drugs, pharmaceuticals, medicaments and nanoparticles.

10. A system for configuring a magnetic field generating device (1) for directioning magnetic substances (300), **characterized by** comprising an image capturing device and a computer device, the image capturing device being electronically connected to the computer device,
the image capturing device being configured to obtain at least one image of at least two dimensions of the body area (500) and locate, in said at least one image of at least two dimensions of the body area (500), a target area (400),
the image capturing device being configured to send said at least one image of at least two dimensions of the target area (400) to the computer device,
the computer device being configured to generate a three-dimensional geometry of the target area (400) from said at least one image of at least two dimensions of the target area (400),
the computer device being further configured to generate a three-dimensional geometry of the magnetic field generating device (1) correlated to the three-dimensional geometry of the target area (400).

11. The system according to claim 10, **characterized in that** the correlation between the magnetic field generating device (1) and the three-dimensional geometry of the target area (400) is carried out by the computer device by simulating a magnetic field gradient for the three-dimensional geometry of the target area (400).

12. The system according to claim 11, **characterized in that** the geometry of the magnetic field generating device (1) is generated from at least one magnet module (11).

13. The system according to claim 12, **characterized in that** the modular association of a plurality of magnets (11) generates the geometry of the magnetic field generating device (1).

14. The system according to any of claims 12 or 13, **characterized in that** the at least one magnet module (11) generates the same magnetic field gradient simulated by the computer device.

15. The system according to claim 14, **characterized in that** the at least one magnet module (11) forms a main magnet (10) of the magnetic field generating device (10).

16. The system according to claim 15, **characterized in that** the magnetic field generating device (1) comprises at least one main magnet (10).

17. The system according to claim 16, **characterized in that** said at least one main magnet (10) may move horizontally in a selective way, upon actuation of at least one handle (201, 202).

18. The system according to claim 17, **characterized in that** the horizontal displacement of said at least one main magnet (10) varies the focus of the magnetic field gradient in the target area (400).

19. The system according to claim 18, **characterized in that**, when said at least one main magnet (10) moves away from the target area (400), the magnetic field gradient of said at least one main magnet (10) is smaller.

20. The system according to claim 18, **characterized in that**, when said at least one main magnet (10) moves closer to the target area (400), the magnetic field gradient of said at least one main magnet (10) is greater.

21. The system according to any of claims 10 to 19, **characterized in that** the magnetic field generating device (1) generates magnetic field with maximum intensity of up to 3 Teslas.
